(19) Europäisches Patentamt
European Patent Office
Office européen des brevets

(11) Publication number: **0 215 736**
**B1**

(12) **EUROPEAN PATENT SPECIFICATION**

(45) Date of publication of patent specification: **29.08.90**

(21) Application number: **86810391.2**

(22) Date of filing: **01.09.86**

(51) Int. Cl.$^5$: **C 07 D 473/12,**
C 07 D 473/08,
C 07 D 473/06, A 61 K 31/52

(54) **Xanthine derivatives, processes for their production and their use as pharmaceuticals.**

(30) Priority: **05.09.85 GB 8522096**
**28.10.85 GB 8526504**

(43) Date of publication of application:
**25.03.87 Bulletin 87/13**

(45) Publication of the grant of the patent:
**29.08.90 Bulletin 90/35**

(84) Designated Contracting States:
**AT BE CH DE FR GB IT LI LU NL SE**

(56) References cited:
**EP-A-0 103 497**
**FR-M- 238**

**Medical Dictionary, 26th Edition, pages 203 and 1355**

**Pharmazeutische Chemie(1982) pp 399, 702**

(73) Proprietor: **SANDOZ AG**
**Lichtstrasse 35**
**CH-4002 Basel (CH)**
(84) **BE CH FR GB IT LI LU NL SE**

(73) Proprietor: **SANDOZ-PATENT-GMBH**
**Humboldtstrasse 3**
**D-7850 Lörrach (DE)**
(84) **DE**

(73) Proprietor: **SANDOZ-ERFINDUNGEN**
**Verwaltungsgesellschaft m.b.H.**
**Brunner Strasse 59**
**A-1235 Wien (AT)**
(84) **AT**

(72) Inventor: **Kis, Zoltan**
**Höhenweg 22**
**CH-4102 Binningen (CH)**
Inventor: **Morley, John**
**Lilienstrasse 64**
**CH-4123 Allschwil (CH)**

**EP 0 215 736 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to novel xanthine derivatives having pharmaceutical, in particular bronchodilator and anti-asthmatic, utility and processes for their production, as well as their use as pharmaceuticals and pharmaceutical compositions comprising them.

FR—M—238 discloses that 8-(p-dimethylamino-benzyl)thiophylline has anti-emetic properties whereas 7-(p-dimethylamino-benzyl)theophylline has no such properties.

More particularly the present invention relates to the compounds of formula I

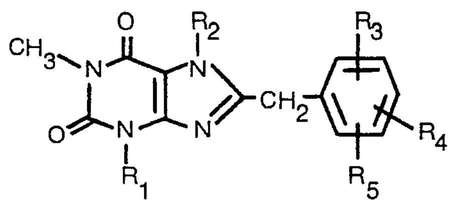

wherein

$R_1$ is $C_{1-4}$alkyl, $C_{3-4}$alkenyl or $C_{3-6}$cycloalkylmethyl,

$R_2$ is $C_{1-4}$alkyl which is substituted by one or more substituents selected from hydroxy, mercapto, $C_{1-4}$alkoxy and $C_{1-4}$alkylthio,

$R_3$ is hydrogen, halogen, hydroxy or $C_{1-4}$alkoxy and $R_4$ and $R_5$ are each independently halogen, hydroxy or $C_{1-4}$alkoxy or together represent a methylene dioxy group ($-O-CH_2-O-$)-bridging adjacent positions on the phenyl ring, and

the physiologically-hydrolysable and -acceptable esters thereof;

Alkyl, alkenyl and alkoxy groups and moieties in the compounds of formula I may be branched or straight chained. When $R_1$ is alkyl this is preferably methyl. When $R_1$ is alkenyl this is preferably allyl. Suitably $R_1$ is methyl, cyclopropylmethyl or allyl.

Groups $R_2$ are substituted by one or more hydroxy, mercapto, $C_{1-4}$alkoxy and/or $C_{1-4}$alkylthio groups. Preferred groups $R_2$ are methyl or $C_{1-4}$alkyl substituted by one or two substituents selected from hydroxy, $C_{1-4}$alkoxy, especially methoxy or $C_{1-4}$alkylthio especially methylthio, for example 2-hydroxyethyl, 2,3-dihydroxypropyl, 2-methoxyethyl, 2-methylthioethyl, 2-hydroxypropyl, 2-hydroxy-2-methylpropyl.

By "halogen" is meant fluorine, chlorine or bromine, especially fluorine or bromine. Preferably $R_3$ is hydrogen, hydroxy or alkoxy, particularly hydroxy or methoxy and $R_4$ and $R_5$ are each independently hydroxy or alkoxy, in particular hydroxy or methoxy or $R_4$ and $R_5$ together are methylenedioxy especially 3,4-methylenedioxy.

In a group of compounds of formula I

$R_1$ is $C_{1-4}$alkyl, $C_{3-4}$alkenyl or cyclopropylmethyl,

$R_2$ is hydroxy substituted $C_{1-4}$alkyl,

$R_3$ is hydrogen, halogen, hydroxy or $C_{1-4}$alkoxy and

$R_4$ and $R_5$ are each independently halogen, hydroxy or $C_{1-4}$alkoxy or together represent a methylenedioxy group ($-O-CH_2-O-$) bridging adjacent positions on the phenyl ring.

Physiologically-hydrolysable and -acceptable esters of the compounds of formula I include both those in which hydroxy or mercapto substituents in $R_2$ are esterified as well as those in which one or more of $R_3$, $R_4$ and $R_5$ is esterified hydroxy. By the term "physiologically-hydrolysable and -acceptable esters" as used herein is meant, esters which are hydrolysable under physiological conditions to yield acids which are themselves physiologically acceptable, i.e which are non-toxic at desired dosage levels. Such esters include those with mono- and di-carboxylic acids, in particular carboxylic acids having 2 to 5 carbon atoms, especially acetic acid.

The compounds of formula I and esters as defined above may exist in free or acid addition salt form. Suitable pharmaceutically acceptable acid addition salts for use in accordance with the present invention include both salts with organic and inorganic acids, e.g. the hydrochlorides and hydrogen fumarates.

Depending on the substitution pattern groups $R_2$ may contain an assymetric carbon atom and thus exist in racemic or isomeric form. Racemic mixtures may be separated into individual isomers in conventional manner. Alternatively optically active starting materials may be employed.

The invention is intended to cover all forms. Unless, otherwise stated the compounds are in racemic form.

In addition to the foregoing the present invention also provides a process for the production of the compounds of formula I and esters as hereinbefore defined and their acid addition salts, which process comprises:

a) For the production of compounds of formula I wherein one or more of $R_2$, $R_3$, $R_4$ and $R_5$ is hydroxy, deprotecting a hydroxy-protected derivative thereof;

b) for the production of compounds of formula I, reacting a compound of formula II

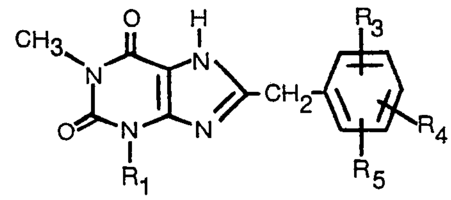

(II)

wherein $R_1$, $R_3$, $R_4$ and $R_5$ have the meanings given for formula I, with a compound of formula III

$$X—R_2 \qquad (III)$$

wherein X is a leaving group and $R_2$ has the meaning given for formula I; or

c) for the production of a physiologically-hydrolysable and -acceptable ester of a compound of formula I esterifying the corresponding compound of formula I so as to introduce an appropriate ester grouping at $R_2$, $R_3$, $R_4$ and/or $R_5$, and recovering the obtained compound of formula I or ester in free or acid addition salt form.

Process step a) may be carried out in accordance with means known in the art for the removal of hydroxy protecting groups, e.g. for the deprotection of benzyl protected hydroxy groups by ether cleavage, for example via hydrogenation in the presence of a Pd/charcoal catalyst as hereinafter described in example 1.

Process step b) may be carried out in accordance with the methods known in the art for example by reaction of II with III in the presence of an acid binding agent, e.g. alkali metal hydroxide, in the presence of an appropriate inert solvent or diluent, e.g. DMPU [1,3-dimethyl-3,4,5,6-tetrahydro-2(1H)-pyrimidinone], e.g. at a temperature of from 5 to 130°C. In formula III, X is suitably a halogen atom, e.g. chlorine or bromine.

Process step c) may also be conducted in entirely conventional manner, e.g. by reaction with an appropriate acid halide or anhydride, suitably in the presence of an acid binding agent such as pyridine, e.g. at a temperature of from ca. 5 to ca. 50°C.

The starting materials for formula II are known (see e.g. Beilsteins Handbuch der Organischen Chemie, 4th. Edition, 26 EIII/IV, p.p. 2332, 2534—6, 2746, 2763—4 and 4012—3) or may be prepared analogously to the known compounds.

The following examples are illustrative of the production of the compounds of the invention:

Example 1

Preparation of 8-(3,4-dimethoxybenzyl)-7-(2-hydroxyethyl)-theophylline [Formula I: $R_1 = CH_3$; $R_2 = HO—(CH_2)_2—$; $R_3 = H$; $R_4 = (4)(CH_3O—$; $R_5 = (3)CH_3O—$]

5.5 g $K_2CO_3$ are added to 10 g 8-(3,4-dimethoxy-benzyl)-theophylline in 150 ml DMPU. After $CO_2$ generation is complete, 3 ml 2-bromoethanol are added and the reaction mixture stirred for 15 hours at 90°C. The formed precipitate is filtered off and the mother liquor freed of DMPU under high vacuum. The residue is dissolved in methylenechloride, filtered and evaporated and the residue re-crystallised from ethanol/ethyl ether to yield the title compound: m.p. = 140—142°C.

The starting material for the above process may be obtained as follows:

100 g 3,4-dimethoxyphenylacetic acid (homoveratric acid) and 106 g 4,5-diamino-1,3-dimethyl-2,6-dioxotetrahydropyrimidinehydrate are finely ground together and mixed and heated in an oil bath to 170°C whereupon the mix melts. The melt is held at 140° for 4 hours and then cooled to 100°C. 40 g NaOH in 750 ml $H_2O$ are then added and the mixture heated for 4½ hours under reflux. The solution is cooled on an icebath and the pH adjusted to 4.2 by addition of 10 N HCl. The crystalline precipitate is collected, washed under suction with $H_2O$ until neutral, washed out three times with 150 ml $C_2H_5OH$/wash and twice with 100 ml ethyl ether/wash and dried at 110°C. The obtained 8-(3,4-dimethoxybenzyl)-theophylline has an m.p. of 242—243°C.

The following compounds may be prepared analogously:

| Example | $R_1$ | $R_2$ | $R_3$ | $R_4$ | $R_5$ | M.P. (°C) |
|---|---|---|---|---|---|---|
| 2 | $CH_3$ | $HO—CH_2—CH_2—$ | H | (2) $CH_3O—$ | (3) $CH_3O—$ | 151—2° |
| 3 | $CH_3$ | $HO—CH_2—CH_2—$ | H | (3) $CH_3O—$ | (5) $CH_3O—$ | 176—7° |
| 4 | allyl | $HO—CH_2—CH_2—$ | H | (3) $CH_3O—$ | (4) $CH_3O—$ | 138—9° |
| 5 | $CH_3$ | 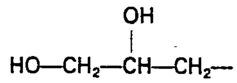 | H | (3) $CH_3O—$ | (4) $CH_3O—$ | 173—4° |
| 6 | 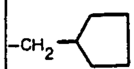 | $HO—CH_2—CH_2—$ | H | (3) $CH_3O—$ | (4) $CH_3O—$ | 113—5° |
| 7 | $CH_3$ | $HO—CH_2—CH_2$ | H | (3) $—O—CH_2—O—$ (4) | | 193—5° |
| 8 | 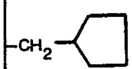 | $CH_2CH_2OCH_3$ | H | (3) $CH_3O$ | (4) $CH_3O$ | 107.5—109° |
| 9 | $CH_3$ | 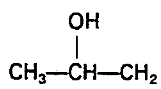 racemate | H | (3) $CH_3O$ | (4) $CH_3O$ | 144—144.5° |
| 10 | allyl | $CH_3OCH_2CH_2$ | H | (3) $CH_3O$ | (4) $CH_3O$ | 76.5—77° |
| 11 | $CH_3$ | 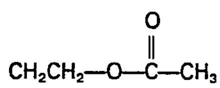 | H | (3) $CH_3O$ | (4) $CH_3O$ | 121—122° |
| 12 | $CH_3$ | $CH_2CH_2SCH_3$ | H | (3) $CH_3O$ | (4) $CH_3O$ | 127—127.6° |
| 13 | $CH_3$ | $CH_2CH_2OCH_3$ | H | (3) $CH_3O$ | (4) $CH_3O$ | 150—150.5° |
| 14 | $CH_3$ | $CH_2CH_2OH$ | H | (2) $CH_3O$ | (4) $CH_3O$ | 186—187° |
| 15 | 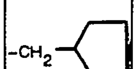 | $CH_2CH_2OH$ | H | (3) $CH_3O$ | (4) $CH_3O$ | 119.5—120° |
| 16 | $CH_3$ | 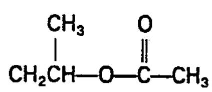 | H | (3) $CH_3O$ | (4) $CH_3O$ | oil |
| 17 | $CH_3$ | 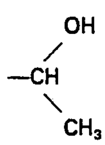 | H | (3) $CH_3O$ | (4) $CH_3O$ | ** 144—144.5° |
| 18 | $CH_3$ | 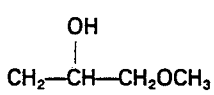 | H | (3) $CH_3O$ | (4) $CH_3O$ | 130—130.5° |

**S(+)isomer $[\alpha_D^{20} = +12.3°$
R(−)isomer $[\alpha_D^{20} = -12.1°$ } c = 1% $CH_2Cl_2$

Example 19

$R_1 = CH_3$; $R_2$ = $—CH_2—C(CH_3)_2OH$; $R_3$ = H; $R_4$ = (4) $CH_3O$; $R_5$ = (3) $CH_3O$; m.p. 173—173.5°.

The compounds of formula I and esters as hereinbefore defined, and their pharmaceutically acceptable acid addition salts (collectively referred to in the description hereinafter as "compounds/salts of the invention") are indicated for use as bronchodilator and anti-asthmatic agents as may be shown in standard test models, e.g. as follows:

1. Spasmolytic activity in airway smooth muscle

1.1 *Tracheal ring relaxation (in Vitro)*

The trachea is excised from freshly sacrificed guinea pig and divided into rings by sectioning at 2 to 3 mm intervals along the sagittal axis. Individual rings are suspended in a bathing medium comprising buffered Kreb's solution gassed with air/carbon dioxide (95:5 V/V). Activity of test compounds is gauged by determination of their ability to relax the ring preparations, which develop spontaneous tone *in vitro.*

"Compounds/salts of the invention" are effective in the above test method at dosages of from about 5 $\times 10^{-6}$ to about $10^{-4}$M.

1.2 *Bronchodilator activity in the Guinea pig*

Guinea pigs are anesthetised with pentabarbital (30 mg/kg i.p.) and phenobarbital (120 mg/kg i.p.) and paralyzed with gallamine (10 mg/kg i.m.). Following cannulation of arterial and venous vessels and trachea, animals are ventilated with an air/oxygen mixture (50:50 v/v) by means of a respirator (1 Hz). Intravenous injection of bombesin at doses of from 200 to 500 ng/kg evokes intense, long-lasting bronchoconstriction. Bronchodilatory effectiveness of test compounds is determined by their ability to inhibit bombesin induced bronchoconstrictor response on i.v. injection.

"Compounds/salts of the invention" inhibit bronchconstriction in the above test method by ca. 50% on administration at dosages of from about 0.5 to about 1.5 mg/kg.

2. Inhibitory action on PAF-induced non-selective airway hyper reactivity

Guinea pigs are prepared and ventilated as described in example 1.2.

On infusion of PAF (600 ng/kg) over 1 hour, test animals respond with intense bronchospasm to a variety of agents (histamine, bombesin and substance P) at doses which induce only marginal changes in airway resistance in animals infused in similar fashion with saline or lyso-PAF.

Effectiveness of test-compound is determined by an ability to reduce or inhibit PAF-induced airway reactivity on prior or concommitant administration.

"Compounds/salts of the invention" are effective in the above test method on administration at doses of from about 1 to about 20 mg/kg i.v.

The "compounds/salts of the invention" are accordingly indicated for use as bronchodilator and anti-asthmatic agents e.g. for the prophylaxis or treatment of asthma, including both allergen and exercise induced asthma, as well as for the treatment of bronchitis, in particular acute bronchitis. An indicated suitable daily dosage is in the range of from about 200 to about 1000 mg e.g. 300 to 1000 mg, conveniently administered in divided doses 2 to 4×/day or in sustained release form. Suitable unit dosage forms accordingly comprise from about 75 to about 500 mg "compound/salt of the invention" together with a pharmaceutically acceptable diluent or carrier therefor.

The "Compounds/salts of the invention" may be administered in similar manner to known standards for use in the recited indications, e.g. orally in oral unit dosage form, e.g. in the form of tablets or capsules.

In accordance with the foregoing the present invention also provides:

i) "Compounds/salts of the invention" for use as pharmaceuticals, e.g. for use as bronchodilator or anti-asthmatic agents, e.g. for use in the prophylaxis or treatment of asthma or for the treatment of bronchitis, in particular acute bronchitis.

ii) A pharmaceutical composition comprising a "compound/salt of the invention" together with a pharmaceutically acceptable diluent or carrier therefor.

Depending on the substitution pattern compounds/salts of the invention may be difficultly soluble particularly in water. Any problems this may cause in administration may be solved by employing suitable galenic techniques or by direct administration of the compound to the lung e.g. in a manner similar to that employed for disodium cromoglycate.

**Claims for the Contracting States: BE CH DE FR GB IT LI LU NL SE**

1. A compound of formula I

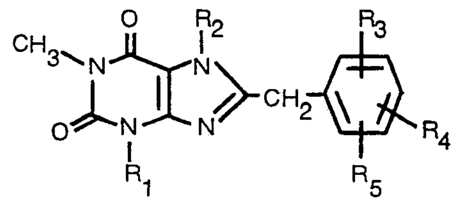

wherein
$R_1$ is $C_{1-4}$alkyl, $C_{3-4}$alkenyl or $C_{3-6}$cycloalkylmethyl,
$R_2$ is $C_{1-4}$alkyl substituted by one or more substituents selected from hydroxy, mercapto, $C_{1-4}$alkoxy and $C_{1-4}$alkylthio,
$R_3$ is hydrogen, halogen, hydroxy or $C_{1-4}$alkoxy and
$R_4$ and $R_5$ are each independently halogen, hydroxy or $C_{1-4}$alkoxy or together represent a methylene dioxy group ($-O-CH_2-O-$)-bridging adjacent positions on the phenyl ring, and
the physiologically-hydrolysable and -acceptable esters thereof.

2. A compound according to claim 1 wherein $R_1$ is $C_{1-4}$alkyl, $C_{3-4}$alkenyl or cyclopropylmethyl, $R_2$ is hydroxy substituted $C_{1-4}$alkyl, $R_3$ is hydrogen, halogen, hydroxy or $C_{1-4}$alkoxy and $R_4$ and $R_5$ are each independently halogen, hydroxy or $C_{1-4}$alkoxy or together represent a methylenedioxy group ($-O-CH_2-O-$)-bridging adjacent positions on the phenyl ring.

3. A compound according to claim 1 which is 8-(3,4-dimethoxybenzyl)-7-(β-methoxy-ethyl)-3-cyclopropylmethyl-xanthine.

4. A compound according to any one of claims 1 to 3 in free form or acid addition salt form.

5. A pharmaceutical composition comprising a compound according to any one of claims 1 to 3 in free form or in pharmaceutically acceptable acid addition salt form together with a pharmaceutically acceptable diluent or carrier thereof.

6. A compound according to any one of claims 1 to 3 in free form or in pharmaceutically acceptable acid addition salt form for use as a pharmaceutical.

7. A compound according to any one of claims 1 to 3 in free form or in pharmaceutically acceptable acid addition salt form for use for the prophylaxis or treatment of asthma or for the treatment of bronchitis in particular acute bronchitis.

8. A process for the production of a compound according to claim 1 which comprises

a) for the production of compounds of formula I wherein one or more of $R_2$, $R_3$, $R_4$ and $R_5$ is hydroxy, deprotecting a hydroxy-protected derivative thereof;

b) for the production of compounds of formula I, reacting a compound of formula II

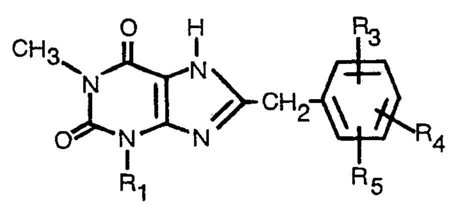

(II)

wherein $R_1$, $R_3$, $R_4$ and $R_5$ have the meanings given for formula I, with a compound of formula III

$$X-R_2 \qquad (III)$$

wherein X is a leaving group and $R_2$ has the meaning given for formula I; or

c) for the production of a physiologically-hydrolysable and -acceptable ester of a compound of formula I esterifying the corresponding compound of formula I so as to introduce an appropriate ester grouping at $R_2$, $R_3$, $R_4$ and/or $R_5$, and recovering the obtained compound of formula I or ester in free or acid addition salt form.

9. A process according to claim 8, comprising separating optically active isomers from any mixture of such isomers obtained in accordance with process steps a), b) or c).

**Claims for the Contracting State: AT**

1. A process for the production of a compound of formula I

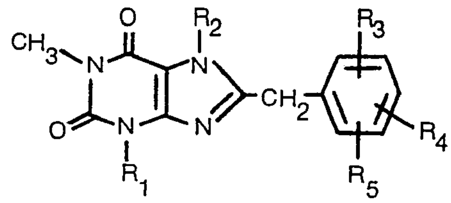

wherein

$R_1$ is $C_{1-4}$alkyl, $C_{3-4}$alkenyl or $C_{3-6}$cycloalkylmethyl,

$R_2$ is $C_{1-4}$alkyl substituted by one or more substituents selected from hydroxy, mercapto, $C_{1-4}$alkoxy, and $C_{1-4}$alkylthio,

$R_3$ is hydrogen, halogen, hydroxy or $C_{1-4}$alkoxy, and

$R_4$ and $R_5$ are each independently halogen, hydroxy or $C_{1-4}$alkoxy or together represent a methylene dioxy group (—O—$CH_2$—O—)-bridging adjacent positions on the phenyl ring, and physiologically hydrolysable and acceptable esters thereof, which process comprises:

a) for the production of compounds of formula I wherein one or more of $R_2$, $R_3$, $R_4$ and $R_5$ is hydroxy, deprotecting a hydroxy-protected derivative thereof;

b) for the production of compounds of formula I, reacting a compound of formula II

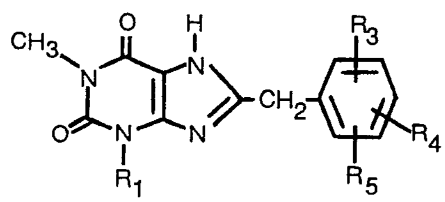

(II)

wherein $R_1$, $R_3$, $R_4$ and $R_5$ have the meanings given for formula I, with a compound of formula III

$$X—R_2 \qquad (III)$$

wherein X is a leaving group and $R_2$ has the meaning given for formula I; or

c) for the production of a physiologically hydrolysable and acceptable ester of a compound of formula I esterifying the corresponding compound of formula I so as to introduce an appropriate ester grouping at $R_2$, $R_3$, $R_4$ and/or $R_5$, and recovering the obtained compound of formula I or ester in free or acid addition salt form.

2. A process according to claim 1 for the production of a compound of formula I as defined in claim 1 wherein $R_1$ is $C_{1-4}$alkyl, $C_{3-4}$alkenyl or cyclopropylmethyl, $R_2$ is hydroxy substituted $C_{1-4}$alkyl, $R_3$ is hydrogen, halogen, hydroxy or $C_{1-4}$alkoxy and $R_4$ and $R_5$ are each independently halogen, hydroxy or $C_{1-4}$alkoxy, or together represent a methylenedioxy group (—O—$CH_2$—O—)-bridging adjacent positions on the phenyl ring.

3. A process according to claim 2 for the production of a compound which is 8-(3,4-dimethoxybenzyl)-7-(β-methoxy-ethyl)-3-cyclopropylmethyl xanthine.

4. A process according to any one of claims 1 to 3 for the production of a compound of formula I as defined in claim 1, in free form or acid addition salt form.

5. A compound of formula I as defined in claim 1, or a physiologically hydrolysable and acceptable ester thereof, obtained by a process according to any one of the preceding claims.

6. A pharmaceutical composition comprising a compound according to claim 5 or a physiologically hyrolysable and acceptable ester thereof, together with a pharmaceutically acceptable diluent or carrier therefor.

7. A process for the preparation of a pharmaceutical composition comprising mixing a compound of formula I

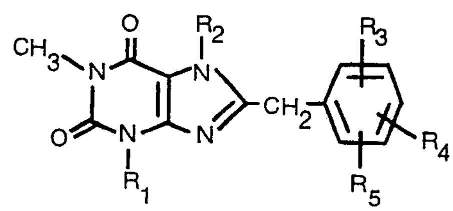

wherein

$R_1$ is $C_{1-4}$alkyl, $C_{3-4}$alkenyl or $C_{3-6}$cycloalkylmethyl,

$R_2$ is $C_{1-4}$alkyl substituted by one or more substituents selected from hydroxy, mercapto, $C_{1-4}$alkoxy and $C_{1-4}$alkylthio,

$R_3$ is hydrogen, halogen, hydroxy or $C_{1-4}$alkoxy, and

$R_4$ and $R_5$ are each, independently, halogen, hydroxy or $C_{1-4}$alkoxy or together represent a methylene dioxy group (—O—$CH_2$—O—)-bridging adjacent positions on the phenyl ring, and physiologically hydrolysable and acceptable esters thereof, with a pharmaceutically acceptable diluent or carrier therefor.

**Patentansprüche für die Vertragsstaaten: BE CH DE FR GB IT LI LU NL SE**

1. Verbindung der Formel I

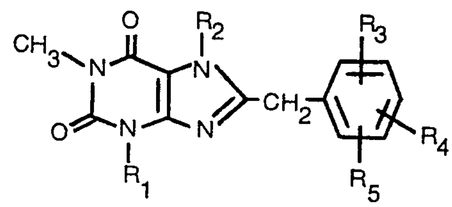

worin

$R_1$ für $C_1$—$C_4$-Alkyl, $C_3$—$C_4$-Alkenyl oder $C_3$—$C_6$-Cycloalkylmethyl steht,

$R_2$ für $C_1$—$C_4$-Alkyl steht, das einfach oder mehrfach durch Hydroxy, Mercapto, $C_1$—$C_4$-Alkoxy oder $C_1$—$C_4$-Alkylthio substituiert ist,

$R_3$ Wasserstoff, Halogen, Hydroxy oder $C_1$—$C_4$-Alkoxy bedeutet und

$R_4$ sowie $R_5$ jeweils unabhängig Halogen, Hydroxy oder $C_1$—$C_4$-Alkoxy oder zusammen eine Methylenedioxygruppe (—O—$CH_2$—O—) bedeuten, welche benachbarte Stellungen am Phenylring überbrückt,

und die physiologisch hydrolysierbaren und physiologisch annehmbaren Ester hiervon.

2. Verbindung nach Anspruch 1, worin $R_1$ für $C_1$—$C_4$-Alkyl, $C_3$—$C_4$-Alkenyl oder Cyclopropylmethyl steht, $R_2$ hydroxysubstituiertes $C_1$—$C_4$-Alkyl bedeutet, $R_3$ Wasserstoff, Halogen, Hydroxy oder $C_1$—$C_4$-Alkoxy ist und $R_4$ und $R_5$ jeweils unabhängig für Halogen, Hydroxy oder $C_1$—$C_4$-Alkoxy stehen oder zusammen eine Methylendioxygruppe (—O—$CH_2$—O—) bilden, durch welche benachbarte Stellungen am Phenylring überbrückt werden.

3. Verbindung nach Anspruch 1, nämlich 8-(3,4-Dimethoxybenzyl)-7-(β-methoxyethyl)-3-cyclopropylmethylxanthin.

4. Verbindung nach einem der Ansprüche 1 bis 3 in freier Form oder in Form eines Säureadditionssalzes.

5. Pharmazeutische Zusammensetzung, enthaltend eine Verbindung nach einem der Ansprüche 1 bis 3 in freier Form oder in Form eines pharmazeutisch annehmbaren Säureadditionssalzes zusammen mit einem pharmazeutisch annhembaren Verdünnungsmittel oder Träger hierfür.

6. Verbindung nach einem der Ansprüche 1 bis 3 in freier Form oder in Form eines pharmazeutisch annehmbaren Säureadditionssalzes zur Verwendung als Pharmazeutikum.

7. Verbindung nach einem der Ansprüche 1 bis 3 in freier Form oder in Form eines pharmazeutisch annehmbaren Säureadditionssalzes zur Verwendung für die prophlaktische oder therapeutische Behandlung von Asthma oder für die Behandlung von Bronchitis, insbesondere von akuter Bronchitis.

8. Verfahren zur Herstellung einer Verbindung nach Anspruch 1, dadurch gekennzeichnet, daß

(a) zur Herstellung von Verbindungen der Formel I, worin einer oder mehrere der Substituenten $R_2$, $R_3$, $R_4$ und $R_5$ für Hydroxy stehen, von einem hydroxygeschützten Derivat hiervon die Schutzgruppen abgespalten werden,

(b) zur Herstellung von Verbindungen der Formel I eine Verbindung der Formel II

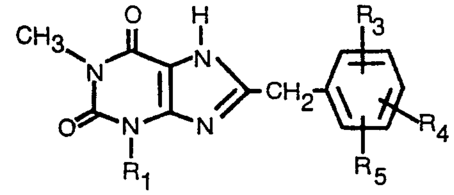

(II)

worin $R_1$, $R_3$, $R_4$ und $R_5$ die bei der Formel I angegebenen Bedeutungen haben, mit einer Verbindung der Formel III

$$X—R_2 \qquad (III),$$

worin X eine Abgangsgruppe ist und $R_2$ die bei der Formel I angegebene Bedeutung besitzt, umgesetzt wird, oder

(c) zur Herstellung eines physiologisch hydrolysierbaren und annehmbaren Esters eine Verbindung der Formel I die entsprechende Verbindung der Formel I unter Einführung einer geeigneten Estergruppe in die Substituenten $R_2$, $R_3$, $R_4$ und/oder $R_5$ verestert wird

und die erhaltene Verbindung der Formel I oder ein Ester hiervon in freier Form oder in Form eines Säureadditionssalzes gewonnen wird.

9. Verfahren nach Anspruch 8, dadurch gekennzeichnet, daß die optisch aktiven Isomeren aus einem Gemisch solcher Isomerer abgetrennt werden, das nach den Verfahrensstufen (a), (b) oder (c) erhalten worden ist.

**Patentansprüche für den Vertragsstaat: AT**

1. Verfahren zur Herstellung einer Verbindung der Formel I

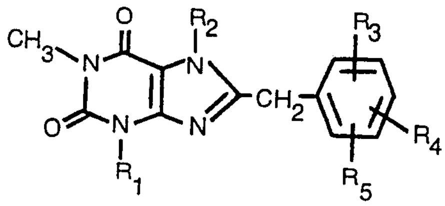

worin

$R_1$ für $C_1$—$C_4$-Alkyl, $C_3$—$C_4$-Alkenyl oder $C_3$—$C_6$-Cycloalkylmethyl steht,

$R_2$ für $C_1$—$C_4$-Alkyl steht, das einfach oder mehrfach durch Hydroxy, Mercapto, $C_1$—$C_4$-Alkoxy oder $C_1$—$C_4$-Alkylthio substituiert ist,

$R_3$ Wasserstoff, Halogen, Hydroxy oder $C_1$—$C_4$-Alkoxy bedeutet und

$R_4$ sowie $R_5$ jeweils unabhängig Halogen, Hydroxy oder $C_1$—$C_4$-Alkoxy oder zusammen eine Methylenedioxygruppe (—O—$CH_2$—O—) bedeuten, welche benachbarte Stellungen am Phenylring überbrückt,

und der physiologisch hydrolysierbaren und physiologisch annehmbaren Ester hiervon, dadurch gekennzeichnet, daß

(a) zur Herstellung von Verbindungen der Formel I, worin einer oder mehrere der Substituenten $R_2$, $R_3$, $R_4$ und $R_5$ für Hydroxy stehen, von einem hydroxygeschützten Derivat hiervon die Schutzgruppen abgespalten werden,

(b) zur Herstellung von Verbindungen der Formel I eine Verbindung der Formel II

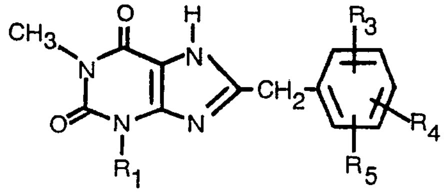

(II)

worin $R_1$, $R_3$, $R_4$ und $R_5$ die bei der Formel I angegebenen Bedeutungen haben, mit einer Verbindung der Formel III

$$X—R_2 \qquad (III),$$

worin X eine Abgangsgruppe ist und $R_2$ die bei der Formel I angegebene Bedeutung besitzt, umgesetzt wird, oder

(c) zur Herstellung eines physiologisch hydrolysierbaren und annehmbaren Esters eine Verbindung der Formel I die entsprechende Verbindung der Formel I unter Einführung einer geeigneten Estergruppe in die Substituenten $R_2$, $R_3$, $R_4$ und/oder $R_5$ verestert wird

und die erhaltene Verbindung der Formel I oder ein Ester hiervon in freier Form oder in Form eines Säureadditionssalzes gewonnen wird.

2. Verfahren nach Anspruch 1 zur Herstellung einer Verbindung der Formel I gemäß Definition von Anspruch 1, worin $R_1$ für $C_1$—$C_4$-Alkyl, $C_3$—$C_4$-Alkenyl oder Cyclopropylmethyl steht, $R_2$ hydroxysubstituiertes $C_1$—$C_4$-Alkyl bedeutet, $R_3$ Wasserstoff, Halogen, Hydroxy oder $C_1$—$C_4$-Alkoxy ist und

$R_4$ und $R_5$ jeweils unabhängig für Halogen, Hydroxy oder $C_1$—$C_4$-Alkoxy stehen oder zusammen eine Methylendioxygruppe (O—$CH_2$—O—) bilden, durch welche benachbarte Stellungen am Phenylring überbrückt werden.

3. Verfahren nach Anspruch 2 zur Herstellung einer Verbindung, welche 8-(3,4-Dimethoxybenzyl)-7-(β-methoxyethyl)-3-cyclopropylmethylxanthin ist.

4. Verfahren nach einem der Ansprüche 1 bis 3 zur Herstellung einer Verbindung der Formel I gemäß Definition von Anspruch 1 in freier Form oder in Form eines Säureadditionsalzes.

5. Verbindung der Formel I gemäß Definition von Anspruch 1 oder eines physiologisch hydrolysierbaren und annhembaren Esters hiervon, dadurch gekennzeichnet, daß sie durch ein Verfahren nach einem der vorhergehenden Ansprüche erhalten worden ist.

6. Pharmazeutische Zusammensetzung, enthaltend eine Verbindung nach Anspruch 5 oder einen physiologisch hydrolysierbaren und annehmbaren Ester hiervon zusammen mit einem pharmazeutisch annehmbaren Verdünnungsmittel oder Träger hierfür.

7. Verfahren zur Herstellung einer pharmazeutischen Zusammensetzung, dadurch gekennzeichnet, daß eine Verbindung der Formel I

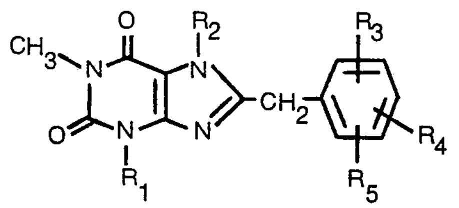

worin

$R_1$ für $C_1$—$C_4$-Alkyl, $C_3$—$C_4$-Alkenyl oder $C_3$—$C_6$-Cycloalkylmethyl steht,

$R_2$ für $C_1$—$C_4$-Alkyl steht, das einfach oder mehrfach durch Hydroxy, Mercapto, $C_1$—$C_4$-Alkoxy oder $C_1$—$C_4$-Alkylthio substituiert ist,

$R_3$ Wasserstoff, Halogen, Hydroxy oder $C_1$—$C_4$-Alkoxy bedeutet und

$R_4$ sowie $R_5$ jeweils unabhängig Halogen, Hydroxy oder $C_1$—$C_4$-Alkoxy oder zusammen eine Methylenedioxygruppe (—O—$CH_2$—O—) bedeuten, welche benachbarte Stellungen am Phenylring überbrückt,

oder ein physiologisch hydrolysierbaren und annehmbarer Ester hiervon mit einem pharmazeutisch annehmbaren Verdünnungsmittel oder Träger hierfür vermischt wird.

**Revendications pour les Etats contractants: BE CH DE FR GB IT LI LU NL SE**

1. Un composé de formule I

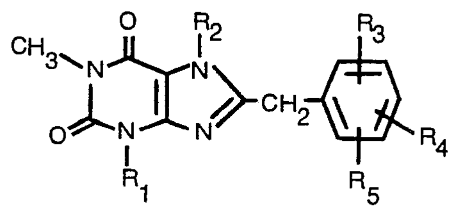

dans laquelle

$R_1$ signifie un groupe alkyle en $C_1$—$C_4$, alcényle en $C_3$—$C_4$ ou (cycloalkyl en $C_3$—$C_6$)-méthyle,

$R_2$ signifie un groupe alkyle en $C_1$—$C_4$ portant un ou plusieurs substituants choisis parmi les groupes hydroxy, mercapto, alcoxy en $C_1$—$C_4$ et alkylthio en $C_1$—$C_4$,

$R_3$ signifie l'hydrogène, un halogène ou un groupe hydroxy ou alcoxy en $C_1$—$C_4$ et

$R_4$ et $R_5$ signifient chacun, indépendamment l'un de l'autre, un halogène ou un groupe hydroxy ou alcoxy en $C_1$—$C_4$ ou représentent ensemble un groupe méthylènedioxy (—O—$CH_2$—O—) reliant des positions adjacentes sur le cycle phényle, et

les esters physiologiquement hydrolysable et physiologiquement acceptables de ces composés.

2. Un composé selon la revendication 1, caractérisé en ce que $R_1$ signifie un groupe alkyle en $C_1$—$C_4$, alcényle en $C_3$—$C_4$ ou cyclopropylméthyle, $R_2$ signifie un groupe alkyle en $C_1$—$C_4$ substitué par un groupe hydroxy, $R_3$ signifie l'hydrogène, un halogène ou un groupe hydroxy ou alcoxy en $C_1$—$C_4$ et $R_4$ et $R_5$ signifient chacun, indépendamment l'un de l'autre, un halogène ou un groupe hydroxy ou alcoxy en $C_1$—$C_4$ ou représentent ensemble un groupe méthylènedioxy (—O—$CH_2$—O—) reliant des positions adjacentes sur le cycle phényle.

3. Un composé selon la revendication 1, qui est la 8-(3,4-diméthoxy-benzyl)-7-(β-méthoxyéthyl)-3-cyclopropylméthyl-xanthine.

4. Un composé selon l'une quelconque des revendications 1 à 3, sous forme libre ou sous forme d'un sel d'addition d'acide.

5. Une composition pharmaceutique, comprenant un composé selon l'une quelconque des revendications 1 à 3, sous forme libre ou sous forme d'un sel d'addition d'acide pharmaceutiquement acceptable, ensemble avec un diluant ou véhicule pharmaceutiquement acceptable.

6. Un composé selon l'une quelconque des revendications 1 à 3, sous forme libre ou sous forme d'un sel d'addition d'acide pharmaceutiquement acceptable, pour l'utilisation comme médicament.

7. Un composé selon l'une quelconque des revendications 1 à 3, sous forme libre ou sous forme d'un sel d'addition d'acide pharmaceutiquement acceptable, pour l'utilisation dans la prophylaxie ou le traitement de l'asthme ou pour le traitement de la bronchite, en particulier de la bronchite aiguë.

8. Un procédé de préparation d'un composé selon la revendication 1, qui comprend

a) pour la préparation des composés de formule I dans laquelle un ou plusieurs des symboles $R_2$, $R_3$, $R_4$ et $R_5$ signifient un groupe hydroxy, la déprotection d'un dérivé de ces composés à groupe hydroxy protégé,

b) pour la préparation des composés de formule I, la réaction d'un composé de formule II

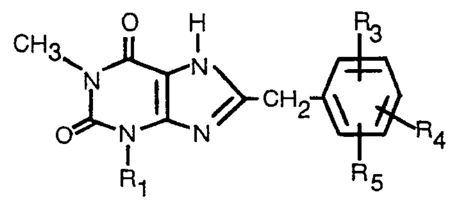

(II)

dans laquelle $R_1$, $R_3$, $R_4$ et $R_5$ ont les significations donnée pour la formule I, avec un composé de formule III

$$X—R_2 \qquad (III)$$

dans laquelle X est un groupe éliminable et $R_2$ a la signification donnée pour la formule I, ou

c) pour la préparation d'un ester physiologiquement hydrolysable et physiologiquement acceptable d'un composé de formule I, l'estérification du composé correspondant de formule I pour introduire un groupement ester approprié sur $R_2$, $R_3$, $R_4$ et/ou $R_5$,

et la récupération du composé obtenu de formule I ou de l'ester sous forme libre ou sous forme d'un sel d'addition d'acide.

9. Un procédé selon la revendication 8, comprenant la séparation des isomères optiquement actifs de tout mélange de tels isomères obtenu selon les étapes a), b) ou c) du procédé.

**Revendications pour l'Etat contractant: AT**

1. Un procédé de préparation d'un composé de formule I

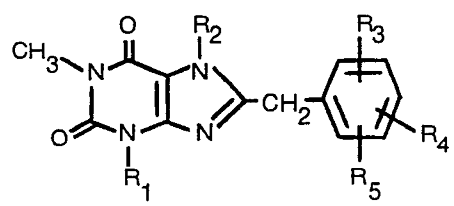

dans laquelle

$R_1$ signifie un groupe alkyle en $C_1$—$C_4$, alcényle en $C_3$—$C_4$ ou (cycloalkyl en $C_3$—$C_6$)-méthyle,

$R_2$ signifie un groupe alkyle en $C_1$—$C_4$ portant un ou plusieurs substituants choisis parmi les groupes hydroxy, mercapto, alcoxy en $C_1$—$C_4$ et alkylthio en $C_1$—$C_4$,

$R_3$ signifie l'hydrogène, un halogène ou un groupe hydroxy ou alcoxy en $C_1$—$C_4$ et

$R_4$ et $R_5$ signifient chacun, indépendamment l'un de l'autre, un halogène ou un groupe hydroxy ou alcoxy en $C_1$—$C_4$ ou représentent ensemble un groupe méthylènedioxy (—O—$CH_2$—O—) reliant des positions adjacentes sur le cycle phényle, et

les esters physiologiquement hydrolysable et physiologiquement acceptables de ces composés, lequel procédé comprend

a) pour la préparation des composés de formule I dans laquelle un ou plusieurs des symboles $R_2$, $R_3$, $R_4$ et $R_5$ signifient un groupe hydroxy, la déprotection d'un dérivé de ces composés à groupe hydroxy protégé,

b) pour la préparation des composés de formule I, la réaction d'un composé de formule II

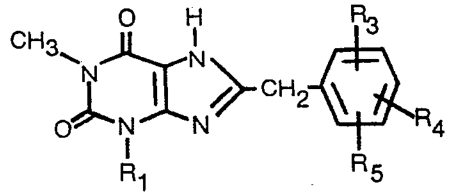

(II)

dans laquelle $R_1$, $R_3$, $R_4$ et $R_5$ ont les signifiations donnée pour la formule I, avec un composé de formule III

$$X—R_2 \qquad \text{(III)}$$

dans laquelle X est un groupe éliminable et $R_2$ a la signification donnée pour la formule I, ou

c) pour la préparation d'un ester physiologiquement hydrolysable et physiologiquement acceptable d'un composé de formule I, l'estérification du composé correspondant de formule I pour introduire un groupement ester approprié sur $R_2$, $R_3$, $R_4$ et/ou $R_5$,

et la récupération du composé obtenu de formule I ou de l'ester sous forme libre ou sous forme d'un sel d'addition d'acide.

2. Un procédé selon la revendication 1 pour la préparation d'un composé de formule I tel que défini à la revendication 1, dans lequel $R_1$ signifie un groupe alkyle en $C_1$—$C_4$, alcényle en $C_3$—$C_4$ ou cyclopropylméthyle, $R_2$ signifie un groupe alkyle en $C_1$—$C_4$ substitué par un groupe hydroxy, $R_3$ signifie l'hydrogène, un halogène ou un groupe hydroxy ou alcoxy en $C_1$—$C_4$ et $R_4$ et $R_5$ signifient chacun, indépendamment l'un de l'autre, un halogène ou un groupe hydroxy ou alcoxy en $C_1$—$C_4$ ou représentent ensemble un groupe méthylènedioxy (—O—$CH_2$—O—) reliant des positions adjacentes sur le cycle phényle.

3. Un procédé selon la revendication 2 pour la préparation d'un composé qui est la 8-(3,4-diméthoxybenzyl)-7-(β-méthoxy-éthyl)-3-cyclopropylméthyl-xanthine.

4. Un procédé selon l'une quelconque des revendications 1 à 3, pour la préparation d'un composé de formule I tel que défini à la revendication 1, sous forme libre ou sous forme d'un sel d'addition d'acide.

5. Un composé de formule I tel que défini à la revendication 1, ou un ester physiologiquement hydrolysable et physiologiquement acceptable de ce composé, obtenu selon un procédé selon l'une quelconque des revendications précédentes.

6. Une composition pharmaceutique comprenant un composé selon la revendication 5 ou un ester physiologiquement hydrolysable et physiologiquement acceptable de ce composé, ensemble avec un diluant ou véhicule pharmaceutiquement acceptable.

7. Un procédé de préparation d'une composition pharmaceutique, comprenant le mélange d'un composé de formule I

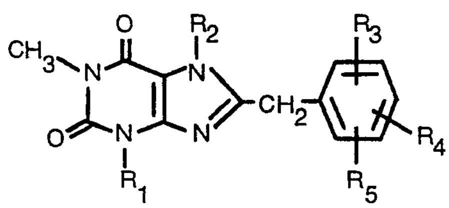

dans laquelle

$R_1$ signifie un groupe alkyle en $C_1$—$C_4$, alcényle en $C_3$—$C_4$ ou (cycloalkyl en $C_3$—$C_6$)-méthyle,

$R_2$ signifie un groupe alkyle en $C_1$—$C_4$ portant un ou plusieurs substituants choisis parmi les groupes hydroxy, mercapto, alcoxy en $C_1$—$C_4$ et alkylthio en $C_1$—$C_4$,

$R_3$ signifie l'hydrogène, un halogène ou un groupe hydroxy ou alcoxy en $C_1$—$C_4$ et

$R_4$ et $R_5$ signifient chacun, indépendamment l'un de l'autre, un halogène ou un groupe hydroxy ou alcoxy en $C_1$—$C_4$ ou représentent ensemble un groupe méthylènedioxy (—O—$CH_2$—O—) reliant des positions adjacentes sur le cycle phényle, et

les esters physiologiquement hydrolysable et physiologiquement acceptables de ces composés, avec un diluant ou véhicule pharmaceutiquement acceptable.